# EUROPÄISCHE PATENTSCHRIFT

(11) **EP 0 564 981 B1**
(45) Veröffentlichungstag und Bekanntmachung des Hinweises auf die Patenterteilung: **02.05.1997**
(21) Anmeldenummer: 93105421.7
(22) Anmeldetag: 01.04.1993
(51) Int. Cl.: C07C 45/54, C07C 49/825, C07C 49/83, C08K 5/13

(54) **Benzophenon-Verbindungen, Verfahren zu deren Herstellung und ihre Verwendung**
Benzophenone compounds, process for their preparation and their use
Composés de benzophénone, procédé pour leur préparation et leur utilisation

(30) Priorität: 04.04.1992 DE 4211419
(43) Veröffentlichungstag der Anmeldung: 13.10.1993
(73) Patentinhaber: HOECHST AKTIENGESELLSCHAFT, 65926 Frankfurt am Main (DE)
(72) Erfinder: Wilharm, Peter, Dr., W-8906 Gersthofen (DE)

(56) Entgegenhaltungen:
- EP-A- 0 057 503
- EP-A- 0 075 390
- WO-A-91/06524
- DE-A- 2 350 180
- DE-B- 1 092 927
- US-A- 3 399 237
- J. CHEM. SOC. C Nr. 1, 1967, Seiten 95 - 99, D.W. CAMERON et al: "The formation of chromanone-type systems via the acylation of derivatives of 2,6-dihydroxyanthracene"

## Beschreibung

Die Erfindung betrifft insbesondere neue 2-Hydroxy-2'-halogen-benzophenon-Verbindungen, Verfahren zu deren Herstellung und ihre Verwendung.

Ortho-Hydroxy-benzophenon-Verbindungen stellen eine kommerziell wichtige Stoffklasse dar und finden beispielsweise Verwendung als Lichtschutzmittel und als Weichermacher für Polymere, als Monomerkomponente für UV-beständige Polyester und als Zwischenprodukte zur Herstellung von Farbstoffen und heterocyclischen Monomeren.

Ortho-Hydroxy-benzophenon-Verbindungen sind durch verschiedene Methoden zugänglich. Grundsätzlich ist es möglich Phenole direkt zu acylieren oder aromatische Phenolester einer Fries'schen-Umlagerung zu unterwefen.

So ist bereits bekannt, daß eine Acylierung von Phenolen, die in para-Stellung bezüglich der phenolischen Gruppe einen Substituenten tragen, mit Säurechloriden unter Anwendung von Aluminiumtrichlorid als Friedel-Crafts-Katalysator zu den entsprechenden Ortho-Hydroxy-benzophenon-Verbindungen reagieren. Nachteilig dabei ist, daß große Mengen Aluminium-Salze als Nebenprodukte anfallen, die entsorgt werden müssen.

Beschrieben wurde die Acylierung von Derivaten des 2,6-Dihydroxyanthracens mit aromatischen Acylierungsmitteln zu Ortho-Hydroxy-benzophenon-Verbindungen (Journal of the Chemical Society, 1 (1967), S.95-99).

Weiterhin ist bekannt, daß eine Acylierung von Phenolen mit freien aromatischen Carbonsäuren in Fluorwasserstoffsäure möglich ist (GB 1 164 046). Solche Reaktionen in Fluorwasserstoffsäure laufen häufig sehr selektiv. Nach Reaktionsende kann die Säure destillativ zurückgewonnen werden. Nachteilig dabei ist, daß die toxische Fluorwasserstoffsäure sehr korrosiv wirkt und die Versuchsdurchführung im technischen Maßstab im allgemeinen sehr aufwendig ist.

Bekannt ist auch, daß sich Phenole mit Carbonsäuren unter Anwendung von Fluoralkansulfonsäuren als Friedel-Crafts-Katalysator zu den entsprechenden Benzophenon-Verbindungen umsetzen lassen (EP 0 075 390). Das beschriebene Verfahren ist an sich attraktiv, leidet aber darunter, daß verhältnismäßig große Mengen der teuren Fluoralkansulfonsäuren angewendet werden müssen. Es wird in der Veröffentlichung erwähnt: "it is preferable to employ at least one mole of fluoralkane sulphonic acid per mole of Ar'-H. In many cases, however, it is useful to employ a large molar excess of the fluoroalkane sulphonic acid..".

Bisacylierte Bisphenol-Verbindungen sind nur in Einzelfällen bekanntgeworden. So ist in F.D. Thomas, A Study of the Double Fries-Rearrangement, J. Amer. Chem. Soc. 80, 5846 (1958) die Herstellung von diacylierten Phenolen unter Anwendung molarer Mengen an Aluminiumtrichlorid beschrieben.
In G.C. Misra, Synthesis of 3,3'-Diacyl-4,4'-Dihydroxybiphenyl, Aust. J. Chem. 25, 1579 (1972) wird die Aluminiumtrichlorid-katalysierte Fries'sche-Umlagerung von Biphenyl-diester beschrieben.
In F.F. Blicke, J. Amer. Chem. Soc. 60, 2283 (1938) wird die Aluminiumtrichlorid-katalysierte Fries'sche-Umlagerung von Phthalsäure-diphenylestern beschrieben.
Allen genannten Verfahren ist die Verwendung von wenigstens molaren Mengen an Aluminiumtrichlorid gemeinsam, was den Nachteil hat, daß bei der Aufarbeitung Aluminiumsalze, die als Nebenprodukte anfallen, entsorgt werden müssen.

Es wird auch die Diacylierung von freien Phenolen und dialkylierten Phenolen mit Benzotrichlorid-Verbindungen beschrieben (DE 1 092 927 und DE 1 114 503). Nachteilig dabei ist, daß die Reaktionsprodukte im allgemeinen. mit tieffarbigen Nebenprodukten verunreigt sind, so daß eine aufwendige Reinigung erforderlich ist.

Es bestand daher die Aufgabe, neue Benzophenon-Verbindungen, insbesondere 2-Hydroxy-2'-halogen-benzophenon-Verbindungen, die sich von Bisphenolen ableiten, bereitzustellen, die einfacher herzustellen sind und die geschilderten Nachteile nicht aufweisen.

Eine weitere Aufgabe bestand darin, ein einfaches, allgemein anwendbares Verfahren zur Herstellung dieser Verbindungen zur Verfügung zu stellen.

Die Erfindung betrifft Benzophenon-Verbindungen der Formel (I) 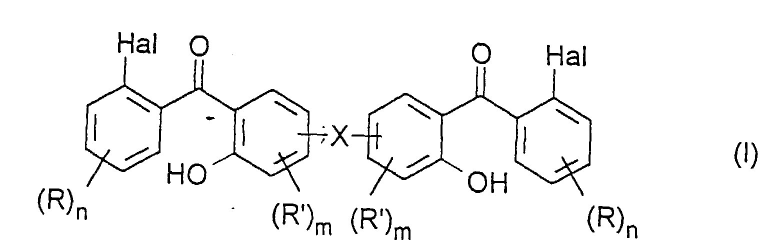 worin Hal für Halogen, vorzugsweise für Chlor- oder Fluor-Gruppen, insbesondere für Chlor-Gruppen, steht, R und R' gleich oder verschieden sind und Wasserstoff, Alkyl-, Alkoxy-, Aryl-, Aryloxy-, Halogen-, Cyano-,Nitro-, Hydroxy-, Amino-, Amido-, N-Alkylamino-, N,N-Dialkylamino-, Carboxyl-, Sulfonsäure-, Alkylsulfonyl-, Arylsulfonyl-, Thiol-Gruppen, benzannelierte Reste, vorzugsweise Wasserstoff, Methyl-, Phenyl-, Methoxy-, Fluor-, Chlor-, Hydroxy-, Amino-, N-Alkylamino-, N,N-Dialkylamino-, Carboxyl-, Sulfonsäure-Gruppen, insbesondere Wasserstoff, Methyl-, Fluor- oder Chlor-Gruppen bedeuten. m und n gleich oder verschieden sind und Null oder eine ganze Zahl, 1 oder 2 bedeuten, und, worin X eine direkte Bindung oder eine -O-, -S-, -SO₂-, -C(CF₃)₂-, -C(CH₃)₂-, Phenylen- oder Dioxyphenylen-Gruppe bedeutet.

Die Erfindung betrifft ferner ein Verfahren zur Herstellung der Verbindungen der Formel (II), 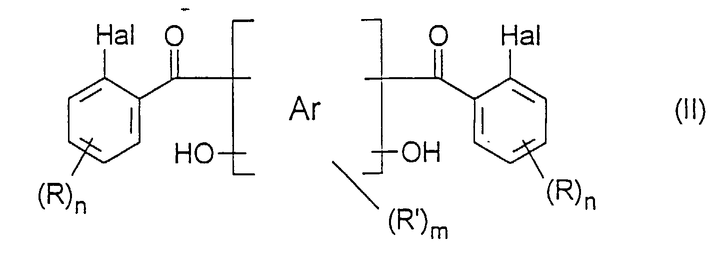 worin Hal, R, R', m und n die bei Formel I genannte Bedeutung haben und wo Ar ein ein-, zwei- oder drei-kerniger, substituierter oder unsubstituierter aromatischer Rest ist, worin die beiden Hydroxy-Gruppen sich in ortho-Stellung zu den jeweiligen Benzophenon-Keto-Gruppen befinden, bei dem ein aromatischer Ester der Formel (III), 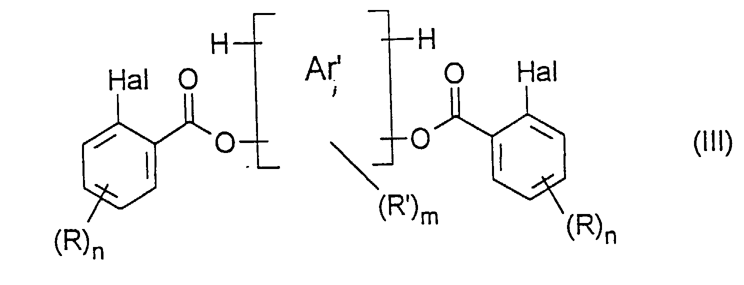 worin Hal, R, R', m, n, die obengenannte bzw. Ar' die für Ar genannte Bedeutung haben und worin Ar'in der jeweiligen ortho-Position zur Oxycarbonyl-Gruppe ein Wasserstoff trägt,
mit oder ohne Lösemittel bei 60 bis 250 °C, vorzugsweise bei 80 bis 220 °C, unter Zusatz einer Mischung bestehend aus 0,01 bis 5 Mol-%, vorzugsweise 0,05 bis 1 Mol-% einer Fluoralkansulfonsäure, vorzugsweise Trifluormethansulfonsäure oder Hexafluorisopropansulfonsäure und 0,1 bis 10 Mol-% vorzugsweise 0,5 bis 5 Mol-% einer Alkansulfonsäure, vorzugsweise Methansulfonsäure oder Methandisulfonsäure, bezogen auf aromatische Ester der Formel (II), wobei ein Mischungsverhältnis von Fluoralkansulfonsäure zu Alkansulfonsäure von 1:1 bis 1:1000, vorzugsweise von 1:5 bis 1:100 gewählt wird, für eine bis 120 Stunden, vorzugsweise 2 bis 48 Stunden dauernde Reaktion unter Rühren erhitzt wird.

Als Vorteil des Verfahrens ist u. a. anzusehen, daß nur katalytische Mengen einer Fluoralkansulfonsäure angewendet werden müssen.

Beispiele für bevorzugte Verbindungen der Formel (I) sind: 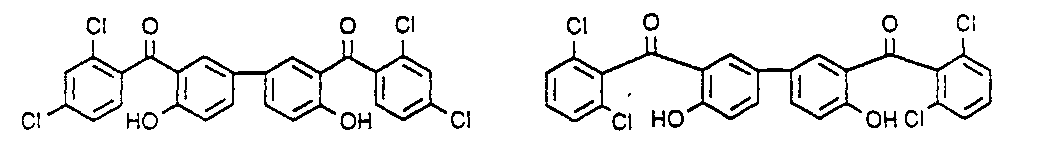 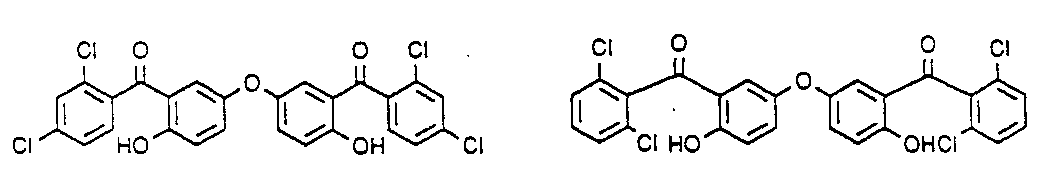 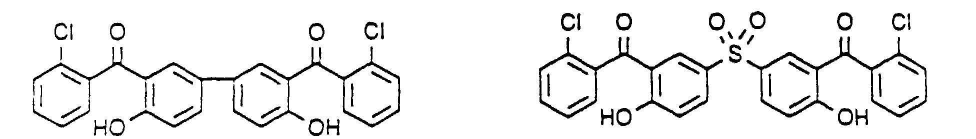 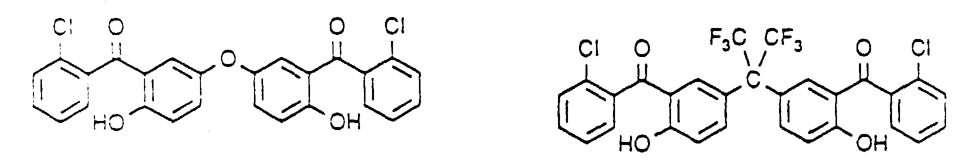 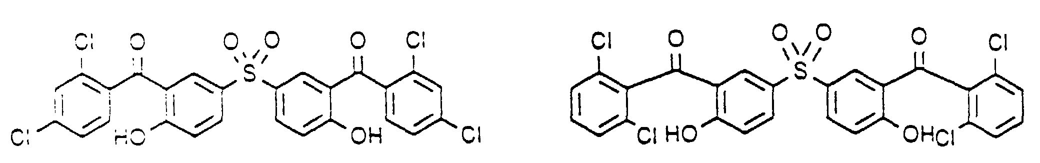 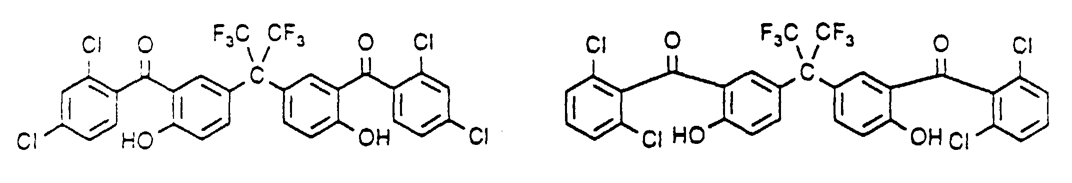 und die entsprechenden kernfluorierten Verbindungen Besonders bevorzugt sind Verbindungen der Formel 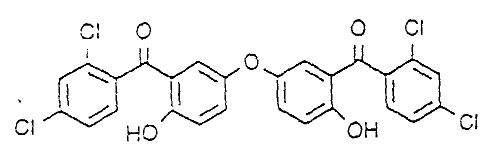 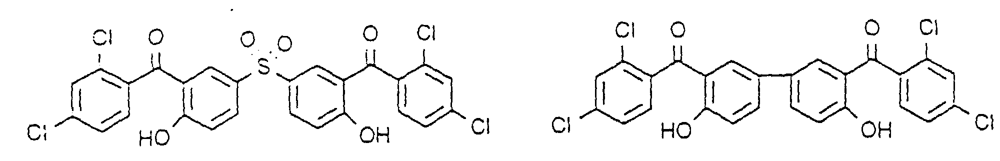 und die entsprechenden kernfluorierten Verbindungen

Die Herstellung der aromatischen Phenolester der Formel (III) und speziell der Phenolester, die als Ausgangsmaterialien zur Herstellung der bevorzugten Benzophenon-Verbindungen der Formel (I) dienen, erfolgt auf an sich bekannte Weise. Hinweise zu den in Frage kommenden Carbonsäure-Verbindungen und Phenol-Verbindungen, aus denen die entsprechenden Phenolester hergestellt werden können und zu geeigneten Lösemitteln, Katalysatoren und zu den Reaktionsbedingungen findet man beispielsweise in H. Henecka, Methoden zur Herstellung von Carbonsäureestern, in Houben-Weyl, Methoden der Organischen Chemie, S. 508ff, Georg Thieme Verlag (1952).

Bei einer bevorzugten Ausführungsform des Verfahrens gemäß der Erfindung wird ohne Lösemittel gearbeitet, wenn die Reaktionsmischung im für die Umlagerung günstigen Temperaturbereich eine Schmelze bildet.

Dabei wird der Phenolester 'in situ' in der Schmelze hergestellt und dann in einem zweiten Schritt ebenfalls in der Schmelze durch Zugabe der Katalysatormischung zum Endprodukt umgewandelt.

Geeignete Methoden zur 'in situ'-Herstellung der Phenolester in der Schmelze für diese Verfahrensvariante sind beispielsweise das sogenannte 'Acetatverfahren' und das 'Schmelzeveresterungsverfahren'.

Beim Acetatverfahren wird ein Phenolacetat zusammen mit der Carbonsäure bei 100 bis 220 °C ohne Zusatz von Lösemittel kondensiert. Die entstehende Essigsäure wird abdestilliert und die Reaktion wird dadurch vervollständigt, daß bei nachlassender Reaktionsgeschwindigkeit schrittweise die Temperatur erhöht und gleichzeitig verminderter Druck angelegt wird.

Bei der Schmelzeveresterungsverfahren wird das Säurechlorid der aromatischen Benzoesäureverbindung zusammen mit dem Phenol bei 60 bis 180 °C unter Abspaltung gasförmiger HCl kondensiert. Die Reaktion kann durch Anlegen von vermindertem Druck oder durch Durchblasen von Inertgas vervollständigt werden.

Für das Verfahren gemäß der Erfindung geeignete Alkansulfonsäuren sind beispielsweise die niedriggliedrigen Alkansulfonsäuren mit endständiger Sulfonsäure-Gruppe, wie Methansulfonsäure, Methandisulfonsäure, Ethansulfonsäure, Propansulfonsäure, Butansulfonsäure.
Bevorzugt wird die kommerziell erhältliche Methansulfonsäure und Methandisulfonsäure.

Für das Verfahren gemäß der Erfindung geeignete Fluoralkansulfonsäuren sind solche, die am alpha-Kohlenstoffatom zur Sulfonsäure-Gruppe wenigstens zwei Fluor-Substituenten tragen.
Beispiel für geeignete Fluoralkansulfonsäuren sind Trifluormethansulfonsäure, Perfluorethansulfonsäure, Perfluorpropan-1-sulfonsäure, Perfluorbutan-1-sulfonsäure, Perfluorpentan-1-sulfonsäure, Perfluorhexan-1-sulfonsäure, Hexafluorisopropyl-sulfonsäure.
Bevorzugt sind Trifluormethansulfonsäure und Hexafluorisopropyl-sulfonsäure.

Die Anwendung der katalytisch wirksamen Mischung aus Fluoralkansulfonsäure und Alkansulfonsäure kann durch vorheriges Mischen der Komponenten und Zugabe zur Reaktionslösung oder durch aufeinanderfolgende Zugabe dieser Säuren zur Reaktionslösung erfolgen.

Das Mischungsverhältnis von Fluoralkansulfonsäure zur Alkansulfonsäure und die angewendete Gesamt-Katalysatorkonzentration (d.h. die Summe an Fluoralkansulfonsäure und Alkansulfonsäure bezogen auf den aromatischen Ester) ist in breiten Grenzen wählbar und richtet sich nach der Reaktivität der eingesetzten Komponenten. So wurde beispielsweise gefunden, daß die Phenolester der 2,6-Dihalogeno-benzoesäuren schon in Anwesenheit sehr geringer Katalysatormengen zu den entsprechenden Benzophenon-Verbindungen umlagert werden. Dagegen erfordern Phenolester, bei denen sich im Phenolteil para-ständig zur Benzoyloxy-Gruppe ein stark elektronenziehender Rest, beispielsweise eine Sulfongruppe, befindet, eine relativ hohe Katalysatorkonzentration mit einem hohen relativen Anteil der Fluoralkansulfonsäure.

Die Temperatur, die für die Umlagerungsreaktion günstig ist und die erforderlichen Reaktionszeiten sind u.a. abhängig von der angewendeten Katalysatorkonzentration und von der Reaktivität der Komponenten. Im Einzelfall können diese Bedingungen auf bekannte Weise optimiert werden. So wurde beispielsweise gefunden, daß 1,3-Bis(2,4-dichlorbenzoyloxy)benzol bei 150 - 180°C glatt zur Benzophenon-Verbindung reagiert, während für den entsprechenden Ester mit der Basis 4,4'-Dihydroxydiphenylsulfon Reaktionstemperaturen von über 210°C erforderlich sind.

Das Verfahren kann bei Normaldruck oder erhöhtem Druck durchgeführt werden. Die Anwendung von Druck kommt dann in Frage, wenn eine der Ausgangsverbindungen oder eine der Katalysatorkomponenten oder gegebenenfalls das verwendete Lösemittel im fraglichen Temperaturbereich der Umlagerung zu flüchtig sind und aus der Reaktionsmischung abdestillieren.

Geeignete Lösemittel sind solche, die unter den angewendeten Reaktionbedingungen stabil sind und die üblicherweise bei elektrophilen Reaktionen angewendet werden.
Beispiele für solche Lösemittel sind ortho-Dichlorbenzol, Chlorbenzol, Tetrachlormethan, 1,1,2,2-Tetrachlorethan, Dichlormethan, Nitromethan, Nitrobenzol, Schwefelkohlenstoff, Sulfolan, Diphenylsulfon.

Vorteilhaft bei vorliegendem Verfahren ist, daß nur geringe Mengen an Katalysator erforderlich sind, die gegebenenfalls nach Reaktionsende leicht abgetrennt und wieder zurückgewonnen werden können.

Wenn eine Rückgewinnung des Katalysatorsystems, beispielsweise aus Umweltschutzgründen oder aus wirtschaftlichen Gründen erwünscht ist, können mehrere Methoden angewendet werden.

Die Abtrennung und Rückgewinnung der Katalysatormischung erfolgt bei der Schmelze-Umlagerung zweckmäßigerweise durch Abdestillation aus der Reaktionsschmelze unter vermindertem Druck oder durch "Vakuum-Strippen".

Bei einer Ausführungsform des Verfahrens, bei dem bei der Umlagerung ein Lösemittel zugesetzt wird, erfolgt die Abtrennung und Rückgewinnung der Katalysatormischung z. B. durch Kristallisation der Benzophenon-Verbindung und anschließender Filtration und destillativer Aufarbeitung der Mutterlauge.

Weiterhin ist, wenn bei einer besonderen Ausführungsform des Verfahrens eine Mischung aus Methansulfonsäure und Trifluormethansulfonsäure als Katalysatormischung verwendet wird, eine Extraktion der erstarrten und anschließend feingemahlenen Schmelze oder bei Verwendung eines Lösemittels, eine Extraktion der Reaktionslösung mit Wasser möglich.

Die Isolierung und gegebenenfalls Reinigung der Benzophenon-Verbindungen aus den Reaktionsmischungen erfolgt auf an sich bekannte Weise.
Solche Verfahrensschritte, wie Fällung, Destillation, Kristallisation, Sublimation usw. sind beispielsweise in EP 0 361 119, in J. VanAllan, J. Org. Chem. 19, 1243 (1954), F.F. Blicke, J. Amer. Chem. Soc. 60, 2283 (1938) näher beschrieben.

Wenn die Reaktionsmischung als Schmelze anfällt, erfolgt die Aufarbeitung vorzugsweise durch Fällung in einem Lösemittel, in dem die jeweilige Benzophenon-Verbindung nur wenig löslich ist und Filtration der ausgefallenen Kristallmasse. Diese Verfahrensweise kann dann vorteilhaft ausgenutzt werden, wenn eventuell anfallende Benzophenon-Isomerengemische getrennt werden sollen.

Die durch das Verfahren gemäß der Erfindung anfallenden Benzophenon-Verbindungen sind meist sehr rein. In Einzelfällen kann eine Nachbehandlung mit Natriumdithionit zweckmäßig sein, um gegebenenfalls gelbliche Verunreinigungen zu entfernen. Nähere Angaben dazu findet man in der Patentschrift GB 1 164 046.

Die neuen ortho-Hydroxybenzophenon-Verbindungen sind wertvolle Zwischenprodukte z.B. zur Herstellung von Xanthonen, Thioxanthonen, Lichtschutzmitteln, Farbstoffen, organischen Pigmenten, Harzen und Polymeren.

Die ortho-Hydroxybenzophenon-Verbindungen sind ferner in polymeren Werkstoffen als temperaturbeständige Lichtschutzmittel und/oder als Stabilisierungsmittel wirksam.

### Beispiele

1) In einen 1-l-Dreihalskolben, ausgestattet mit einem Innenthermometer, mechanischem Rührer, Rückflußkühler mit Blasenzähler sowie thermostatisierter Ölbadheizung wurden folgende Rezepturbestandteile eingewogen:
100 g Resorcin und 380,5 g 2,4-Dichlor-benzoylchlorid. Die Reaktionsmischung wurde unter Rühren auf 90 °C Badtemperatur aufgeheizt. Dabei setzte unter Schäumen eine starke HCl-Entwicklung ein. Es wurde solange bei dieser Temperatur gerührt bis die HCl-Entwicklung deutlich nachließ (ca. 30 Minuten), dann die Badtemperatur auf 160 °C gesteigert und weitere 90 Minuten bei dieser Temperatur gerührt.
Anschließend wurde eine Mischung aus 1 ml Trifluormethansulfonsäure und 20 ml Methansulfonsäure zugegeben und solange gerührt, bis die Kristallisation der Reaktionsmasse einsetzte. Der Rührer wurde abgeschaltet, die Badtemperatur auf 180 °C erhöht und die Reaktionsmasse noch 3 Stunden bei dieser Temperatur gehalten.
Zur Aufarbeitung wurden 300 ml Dichlorbenzol zugegeben, um die erstarrte Reaktionsmasse wieder zu verflüssigen und dann die heiße Lösung in 2 l Methanol gefällt.
Die methanolische Lösung wurde auf 5 °C gekühlt und die ausgefallene Kristallmasse abgesaugt. Anschließend wurde mit 2 X 400 ml kaltem Methanol aufgeschlämmt und abermals abgesaugt. Nach der Trocknung bei 50 °C unter vermindertem Druck erhielt man 384 g gelblich gefärbte Kristalle vom Schmelzpunkt 209° C.

Das ¹³C-NMR-Spektrum gemessen in [D₆]-DMSO zeigte folgende Signale: ¹³C-NMR (CDCl₃, 90 MHz): 197,2 (C=O), 169,7, 142,7, 137,3, 134,6, 131,9, 129,7, 129,5, 127,1, 113,5, 105,2 ppm und steht in Einklang mit folgender Struktur:

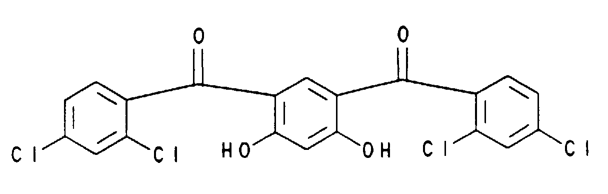

Aus der methanolischen Mutterlauge kann das entsprechende 2,4-Isomere gewonnen werden. Dazu wurde die Methanol-Mutterlauge eingeengt, die ausgefallene Kristallmasse abgesaugt und mit Hilfe der Säulenchromatographie gereinigt (Kieselgel, Laufmittel Toluol).
Das ¹³C-NMR-Spektrum gemessen in CDCl₃ zeigte folgende Signale:
196,5 (C=O), 191,4 (C=O), 164,1, 162,9, 137,5, 136,7, 136,5, 135,7, 135,5, 132,2, 132,0, 130,6, 130,1, 129,3, 127,6, 114,8, 112,2, 108,9 ppm und steht in Einklang mit folgender Struktur:

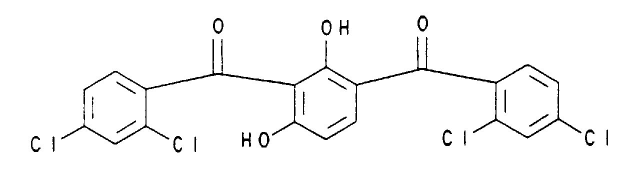

Beim dünnschichtchromatographischen Nachweis zeigen die Isomere folgende Werte (Kieselgel mit Fluoreszenzindikator 254 nm, Laufmittel Toluol):
R_{F} 4,6-Bis-(2',4'-dichlorbenzoyl)-resorcin: 0,75
R_{F} 2,4-Bis-(2',4'-dichlorbenzoyl)-resorcin: 0,90
Das Isomerenverhältnis 4,6-Isomer/2,4-Isomer betrug 95:5.

Durch Erniedrigung der Temperatur bei der Umlagerung kann das Isomerenverhältnis beeinflußt werden.
Bei Wiederholung des Versuches in 200 ml 1,2-Dichlorbenzol bei einer Umlagerungstemperatur von 100 °C während 12 Stunden erhält man ein Isomerenverhältnis 4,6-Isomer/2,4-Isomer von 76:24.

2) 4,6-Bis-(2'-dichlorbenzoyl)-1,3-dihydroxy-benzol:
In einen 1-l-Dreihalskolben, ausgestattet mit einem Innenthermometer, mechanischem Rührer und Claisenbrücke sowie thermostatisierter Ölbadbeheizung wurden folgende Rezepturbestandteile eingewogen: 176,3 g Resorcindiacetat und 284,3 g 2-Chlor-benzoesäure. Die Reaktionsmischung wurde unter Rühren langsam auf 160° C Badtemperatur aufgeheizt. Innerhalb 60 Minuten destillierte der Großteil der bei der Veresterung freiwerdenden Essigsäure ab. Anschließend wurde verminderter Druck angelegt und die Temperatur auf 180 °C gesteigert. Dann wurde mit Inertgas belüftet und die Temperatur auf 160 °C abgesenkt.
Anschließend wurde eine Mischung aus 0,5 ml Hexafluoroisopropylsulfonsäure und 10 ml Methansulfonsäure zugegeben und 60 Minuten bei dieser Temperatur und anschließend 12 Stunden bei 180° C gerührt.
Zur Aufarbeitung wurde die Schmelze in einem dünnen Strahl unter gutem Rühren langsam in 3 l Aceton gefällt.
Die Aceton-Lösung wurde auf 5° C gekühlt und die ausgefallene Kristallmasse abgesaugt, mit 2 X 400 ml kaltem Methanol aufgeschlämmt, abgesaugt und bei vermindertem Druck getrocknet.

Das ¹³C-NMR-Spektrum gemessen in [D₆]-DMSO zeigte folgende Signale: 195,3 (C=O), 166,9, 140,0, 137,9, 131,5, 129,5, 129,4, 128,7, 127,1, 115,2, 103,8 ppm.

3) In einen 1-l-Dreihalskolben, ausgestattet mit einem Innenthermometer, mechanischem Rührer sowie thermostatisierter Ölbadheizung wurden folgende Rezepturbestandteile eingewogen: 100 g Biphenyl-di(2,4-dichlorbenzoesäure)ester, 500 ml Dichlorbenzol, 5 ml Trifluormethansulfonsäure und 5 ml Ethansulfonsäure. Die Lösung wurde 16 Stunden unter Rückfluß gerührt. Anschließend kühlte man die Lösung auf 0 °C ab, filtrierte die ausgefallene Kristallmasse ab und trocknete unter vermindertem Druck bei 100° C. Ausbeute 94 g gelbe Kristalle.
Das ¹³C-NMR-Spektrum gemessen in [D₆]-DMSO zeigte folgende Signale: 195,3 (C=O), 166,9, 140,0, 137,9, 131,5, 129,5, 129,4, 128,7, 127,1, 115,2, 103,8 ppm.

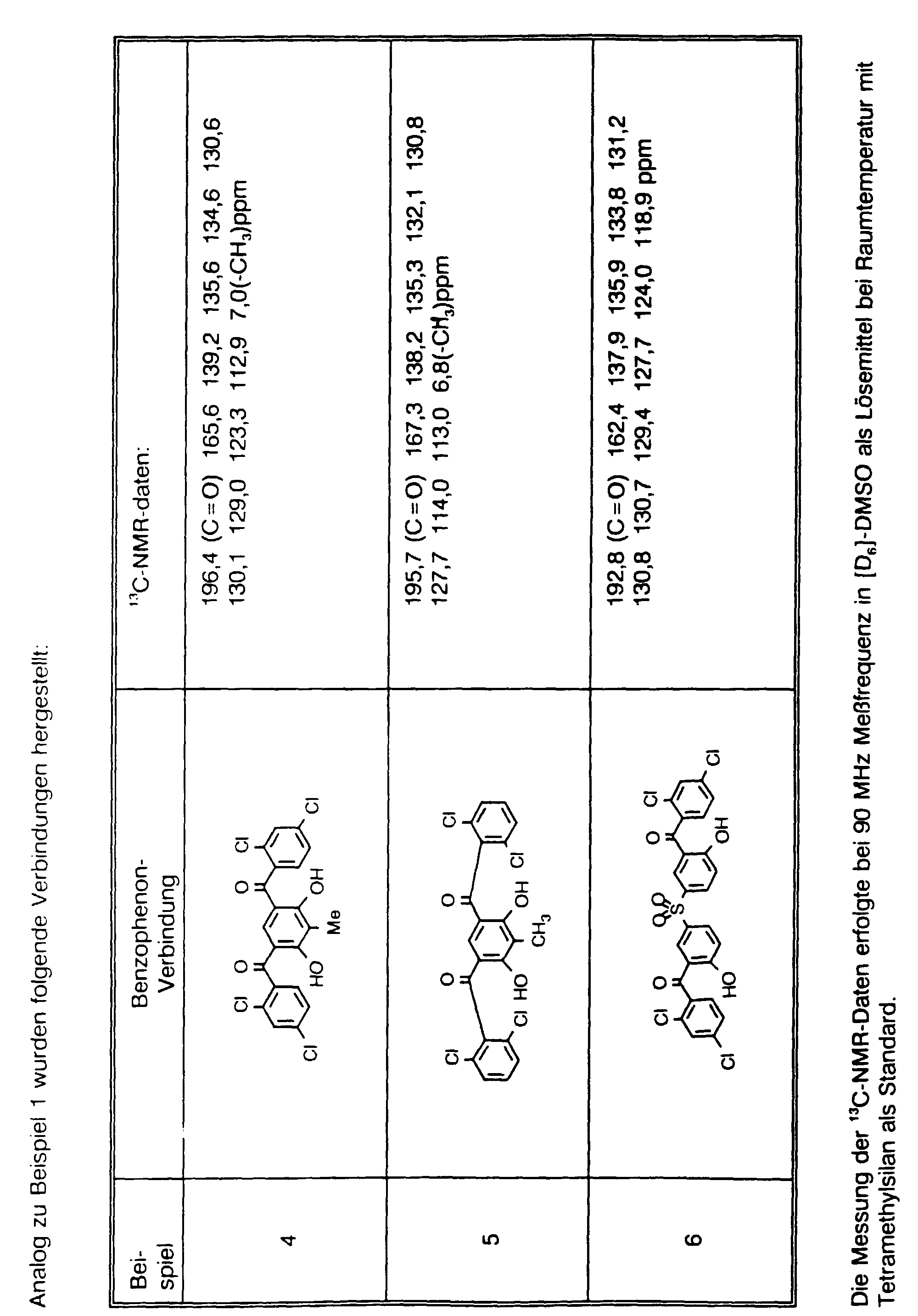

## Patentansprüche

1. Benzophenon-Verbindungen der Formel (I) 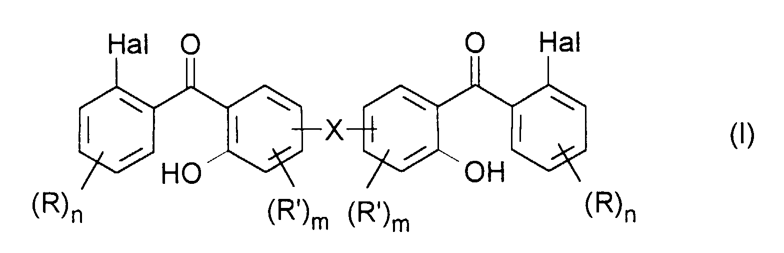 worin Hal für Halogen, R und R' gleich oder verschieden sind und Wasserstoff, Alkyl-, Alkoxy-, Aryl-, Aryloxy-, Halogen-, Cyano-, Nitro-, Hydroxy-, Amino-, Amido-, N-Alkylamino-, N,N-Dialkylamino-, Carboxyl-, Sulfonsäure-, Alkylsulfonyl-, Arylsulfonyl-, Thiol-Gruppen, benzannelierte Reste bedeuten, m und n gleich oder verschieden sind und Null oder eine ganze Zahl 1 oder 2 bedeuten und worin X eine direkte Bindung oder eine -O-, -S-, -SO₂-, -C(CF₃)₂-, -C(CH₃)₂-, Phenylen- oder Dioxyphenylen-Gruppe bedeutet.

2. Benzophenon-Verbindungen nach Anspruch 1, dadurch gekennzeichnet, daß Hal für Chlor- oder Fluor-Gruppen, insbesondere für Chlor-Gruppen, steht, R und R' Wasserstoff, Methyl-, Phenyl-, Methoxy-, Fluor-, Chlor-, Hydroxy-, Amino-, N-Alkylamino-, N,N-Dialkylamino-, Carboxyl-, Sulfonsäure-Gruppen, insbesondere Wasserstoff, Methyl-, Fluor- oder Chlor-Gruppen bedeuten.

3. Verfahren zur Herstellung von Benzophenon-Verbindungen der Formel (II), 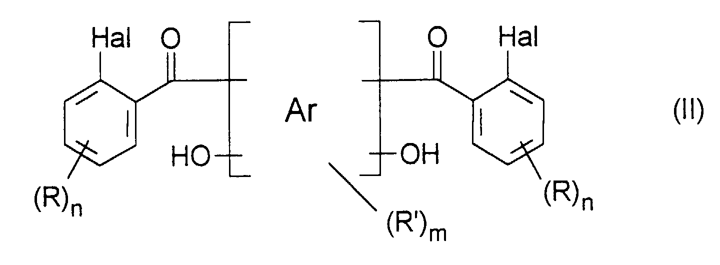 worin Hal, R, R', m und n die bei Formel I genannte Bedeutung haben und wo Ar ein ein-, zwei- oder drei-kerniger, substituierter oder unsubstituierter aromatischer Rest ist, worin die beiden Hydroxy-Gruppen sich in ortho-Stellung zu den jeweiligen Benzophenon-Keto-Gruppen befinden,
dadurch gekennzeichnet, daß ein aromatischer Ester der Formel (III), 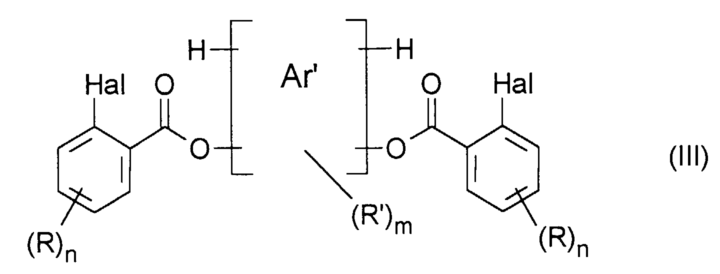 worin Hal, R, R', m, n die genannte bzw. Ar' die dort für Ar genannte Bedeutung haben und worin Ar' in der jeweiligen ortho-Position zur Oxycarbonyl-Gruppe ein Wasserstoff trägt,
mit oder ohne Lösemittel bei 60 bis 250° C, unter Zusatz einer Katalysator-Mischung bestehend aus 0,01 bis 5 Mol-% einer Fluoralkansulfonsäure, und 0,1 bis 10 Mol-% einer Alkansulfonsäure, bezogen auf aromatische Ester der Formel (III), wobei ein Mischungsverhältnis von Fluoralkansulfonsäure zu Alkansulfonsäure von 1:1 bis 1:1000 gewählt wird, für eine bis 120 Stunden **dauernde Reaktion** unter Rühren erhitzt und der Katalysator gegebenenfalls nach Reaktionsende abgetrennt wird.

4. Verfahren nach Anspruch 3, dadurch gekennzeichnet, daß die Umsetzung bei 80 bis 220° C unter Zusatz von 0,05 bis 1 Mol-% einer Fluoralkansulfonsäure, vorzugsweise Trifluormethansulfonsäure oder Hexafluorisopropansulfonsäure und 0,5 bis 5 Mol-% einer Alkansulfonsäure, vorzugsweise Methansulfonsäure bei einem Mischungsverhältnis von Fluoralkan- zu Alkansulfonsäure von 1:5 bis 1:100 für 2 bis 48 Stunden erfolgt.

5. Verfahren nach Anspruch 3 oder 4, dadurch gekennzeichnet, daß die Umsetzung bei Normaldruck oder erhöhtem Druck durchgeführt wird.

6. Verfahren nach einem oder mehreren der Ansprüche 3 bis 5, dadurch gekennzeichnet, daß als Lösemittel ortho-Dichlorbenzol, Chlorbenzol, Tetrachlormethan, 1,1,2,2-Tetrachlorethan, Dichlormethan, Nitromethan, Nitrobenzol, Schwefelkohlenstoff, Sulfolan oder Diphenylsulfon eingesetzt werden.

7. Verwendung der Benzophenon-Verbindungen nach Anspruch 1 als Zwischenprodukte zur Herstellung von Xanthonen, Thioxanthonen, Lichtschutzmitteln, Farbstoffen, organischen Pigmenten, Harzen und Polymeren sowie als Lichtschutzmittel und/oder Stabilisierungsmittel in polymeren Werkstoffen.

## Claims

1. A benzophenone compound of the formula (I) 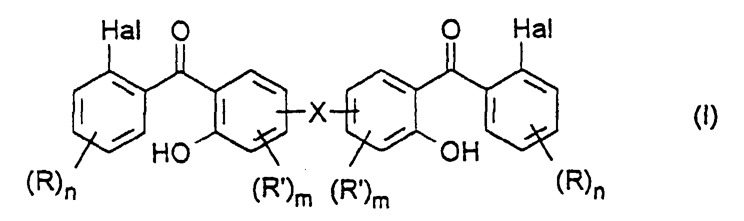 in which Hal is halogen, R and R' are identical or different and are hydrogen, alkyl, alkoxy, aryl, aryloxy, halogen, cyano, nitro, hydroxyl, amino, amido, N-alkylamino, N,N-dialkylamino, carboxyl, sulfonic acid, alkylsulfonyl, arylsulfonyl or thiol groups or benzo-fused radicals, m and n are identical or different and are zero or the integer 1 or 2, and in which X is a direct bond or an -O-, -S-, -SO₂-, -C(CF₃)₂-, -C(CH₃)₂-phenylene or dioxyphenylene group.

2. A benzophenone compound as claimed in claim 1, in which Hal is chlorine or fluorine groups in particular, chlorine groups, and R and R' are hydrogen or methyl, phenyl, methoxy, fluoro, chloro, hydroxylamino, N-alkylamino, N,N-dialkylamino, carboxyl or sulfonic acid groups, in particular hydrogen or methyl, fluorine or chlorine groups.

3. A process for the preparation of a benzophenone compound of the formula (II) 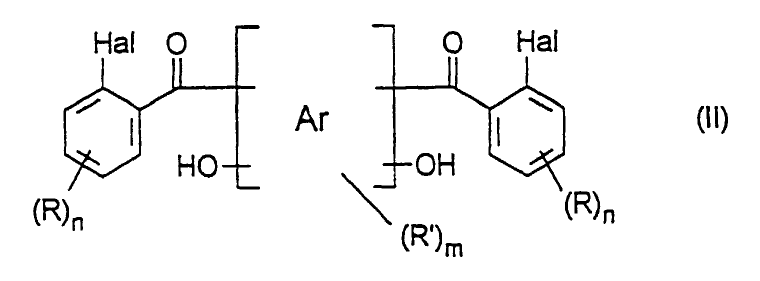 in which Hal, R, R', m and n have the meaning given in the case of formula I and where Ar is a mono-, di- or trinuclear, substituted or unsubstituted aromatic radical, in which the two hydroxyl groups are in the ortho-position relative to the particular benzophenone keto groups, which comprises heating an aromatic ester of the formula (III) 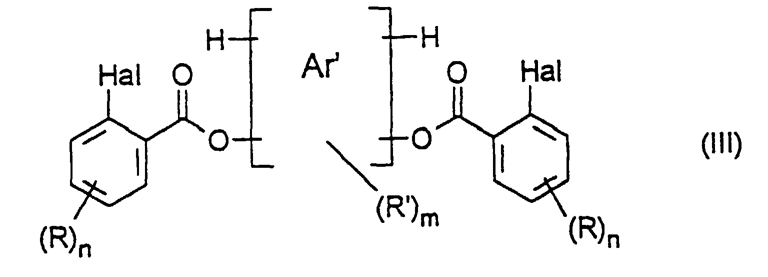 in which Hal, R, R', m and n have the meaning given and Ar' has the meaning given there for Ar, and in which Ar' carries a hydrogen in the particular ortho-position relative to the oxycarbonyl group,
at 60 to 250°C, with or without a solvent, with the addition of a catalyst mixture comprising 0.01 to 5 mol % of a fluoroalkanesulfonic acid and 0.1 to 10 mol % of an alkanesulfonic acid, based on the aromatic ester of the formula (III), a mixing ratio of fluoroalkanesulfonic acid to alkanesulfonic acid of 1:1 to 1:1000 being chosen, for a reaction lasting up to 120 hours, while stirring, and, if appropriate, separating off the catalyst after the end of the reaction.

4. The process as claimed in claim 3, wherein the reaction is carried out at 80 to 220°C with the addition of 0.05 to 1 mol % of a fluoroalkanesulfonic acid, preferably trifluoromethanesulfonic acid or hexafluoroisopropanesulfonic acid, and 0.5 to 5 mol % of an alkanesulfonic acid, preferably methanesulfonic acid, at a mixing ratio of fluoroalkane- to alkanesulfonic acid of 1:5 to 1:100 for 2 to 48 hours.

5. The process as claimed in claim 3 or 4, wherein the reaction is carried out under normal pressure or increased pressure.

6. The process as claimed in one or more of claims 3 to 5, wherein ortho-dichlorobenzene, chlorobenzene, carbon tetrachloride, 1,1,2,2-tetrachloroethane, dichloromethane, nitromethane, nitrobenzene, carbondisulfide, sulfolane or diphenyl sulfone is employed as the solvent.

7. The use of a benzophenone compound as claimed in claim 1 as an intermediate for the preparation of xanthones, thioxanthones, light stabilizers, dyestuffs, organic pigments, resins and polymers and as a light stabilizer and/or stabilizing agent in polymeric materials.

## Revendications

1. Composés de type benzophénone de formule (I) 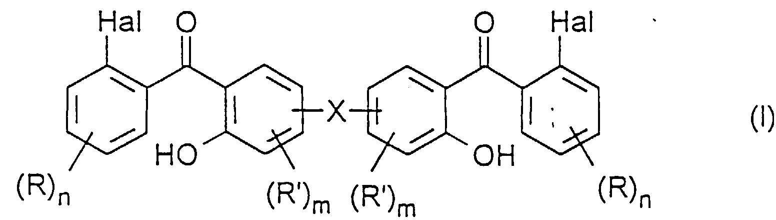 dans laquelle Hal représente halogène, R et R' sont identiques ou différents et représentent l'hydrogène, des groupes alkyle, alcoxy, aryle, aryloxy, halogène, cyano, nitro, hydroxy, amino, amido, N-alkylamino, N,N-dialkylamino, carboxyle, acide sulfonique, alkylsulfonyle, arylsulfonyle, thiol, des radicaux cyclisés sur le benzène, m et n sont identiques ou différents et sont zéro ou représentent un nombre entier, 1 ou 2, et dans laquelle X représente une liaison directe ou un groupe -O-, -S-, SO₂-, -C(CF₃)₂-, -C(CH₃)₂-, phénylène ou dioxyphénylène.

2. Composés de type benzophénone selon la revendication 1, caractérisés en ce que Hal représente des groupes chlore ou fluor, en particulier des groupes chlore, R et R' représentent l'hydrogène, des groupes méthyle, phényle, méthoxy, fluor, chlore, hydroxy, amino, N-alkylamino, N,N-dialkylamino, carboxyle, acide sulfonique, en particulier l'hydrogène, des groupes méthyle, fluor ou chlore.

3. Procédé de préparation de composés de type benzophénone de formule (II), 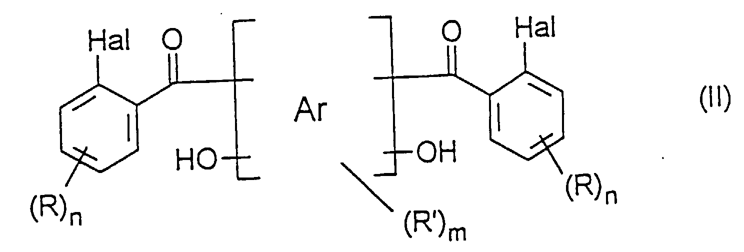 dans laquelle Hal, R, R', m et n ont la signification mentionnée pour la formule I et où Ar est un radical aromatique substitué ou non substitué, mono-, bi- ou tricyclique, dans lequel les deux groupes hydroxy se trouvent en position ortho par rapport aux groupes céto de benzophénone respectifs,
caractérisé en ce qu'on chauffe sous agitation, pendant une durée de réaction allant jusqu'à 120 heures, un ester aromatique de formule (III), 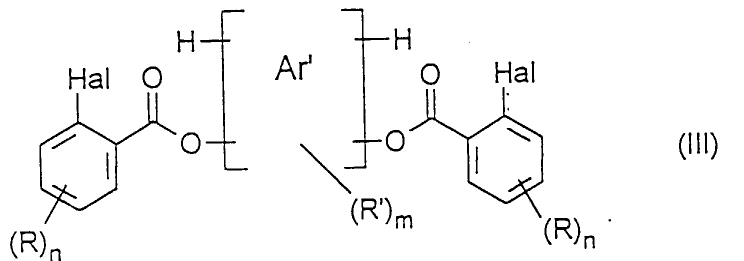 dans laquelle Hal, R, R', m, n ont la signification mentionnée, respectivement Ar' a la signification mentionnée ici pour Ar et dans laquelle Ar' porte un hydrogène dans la position ortho respective par rapport au groupe oxycarbonyle,
avec ou sans solvant à 60 jusqu'à 250°C, en présence d'un mélange catalytique constitué de 0,01 à 5 mol % d'un acide fluoroalcanesulfonique et de 0,1 à 10 mol % d'un acide alcanesulfonique, par rapport à l'ester aromatique de formule (III), un rapport de mélange de l'acide fluoroalcanesulfonique à l'acide alcanesulfonique de 1:1 à 1:1000 étant choisi et en ce qu'on sépare éventuellement le catalyseur après la fin de la réaction.

4. Procédé selon la revendication 3, caractérisé en ce que la réaction a lieu à 80 jusqu'à 220°C en présence de 0,05 à 1 mol % d'un acide fluoroalcanesulfonique, de préférence l'acide trifluorométhanesulfonique ou l'acide hexafluoroisopropanesulfonique et de 0,5 à 5 mol % d'un acide alcanesulfonique, de préférence l'acide méthanesulfonique à un rapport de mélange de l'acide fluoroalcanesulfonique à l'acide alcanesulfonique de 1:5 à 1:100, pendant 2 à 48 heures.

5. Procédé selon la revendication 3 ou 4, caractérisé en ce que la réaction est effectuée à la pression normale ou à une pression augmentée.

6. Procédé selon l'une ou plusieurs des revendications 3 à 5, caractérisé en ce que l'ortho-dichlorobenzène, le chlorobenzène, le tétrachlorométhane, le 1,1,2,2-tétrachloroéthane, le dichlorométhane, le nitrométhane, le nitrobenzène, le sulfure de carbone, le sulfolane ou la diphénylsulfone sont utilisés comme solvant.

7. Utilisation des composés de type benzophénone selon la revendication 1 comme produits intermédiaires pour la préparation de xanthones, thioxanthones, agents de protection contre la lumière, colorants, pigments organiques, résines et polymères, ainsi que comme agents de protection contre la lumière et/ou stabilisants dans des matériaux polymères.
